# EUROPEAN PATENT APPLICATION

(11) **EP 4 450 124 A1**
(43) Date of publication of application: **23.10.2024**
(21) Application number: 23168409.3
(22) Date of filing: 18.04.2023
(51) Int. Cl.: A61N 2/02, A61N 2/00

(54) **COIL ARRANGEMENT AND DEVICE FOR TRANSCRANIAL MAGNETIC STIMULATION**

(71) Applicant: Brightmind.AI GmbH, 1010 Wien (AT)
(72) Inventor: Kühne, André, 13129 Berlin (DE); Gilhooley, Séamus, 1180 Vienna (AT); Gerbert, Tamara, 1090 Vienna (AT); Lerchbammer-Kreith, Florian, 1010 Vienna (AT)
(74) Representative: Heeschen Pültz Patentanwälte PartGmbB

(57) **Abstract**

The invention relates to a coil arrangement (100) for transcranial magnetic stimulation and a device (200) comprising such a coil arrangement (100). The coil arrangement (100) comprising a first coil (110) having a first plurality of windings, the first plurality of windings comprising at least an inner winding (111) and an outer winding (112); a second coil (120) having a second plurality of windings, the second plurality of windings comprising at least an inner winding (121) and an outer winding (122), wherein the first and second coils (110, 120) are arranged adjacent to each other along a first predetermined surface (140), wherein the outer winding (112, 122) surrounds at least partially the inner winding (111, 121) of the respective first and second coils (110, 120) along the first predetermined surface (140), wherein adjacent sections of the outer windings (112, 122) of the first and second coils (110, 120) are at least partially arranged parallel to each other.

## Description

The invention relates to a coil arrangement for transcranial magnetic stimulation and a device comprising such a coil arrangement.

Transcranial magnetic stimulation, TMS is a non-invasive technique that is used for numerous research and therapeutic applications, including the study of normal and pathological brain function and the treatment of neurological and psychiatric disorders. TMS uses brief, intense pulses of electric current delivered to a coil placed on the subject's head to generate an electric field in the brain via electromagnetic induction. The induced electric field modulates the neural transmembrane potentials and, thereby, neural activity. The location of the stimuli depends on the induced electrical field, which in turn is dependent on the coil geometry and placement.

One of the main problems that exists in current TMS coil design is to successfully translate simulated minimum-energy or loss designs into a real-world physical device due to locally high current densities and corresponding high loss power densities. For instance, the physical realization of most simulation-based designs would require extremely thin wires, thus leading to very high losses since the available surface is not densely covered with thin wires. In reality, the maximum usable wire diameter is, however, directly tied to the maximum local current density. In addition, TMS may lead to a heating of the coil design and said heating must be restricted to a certain temperature threshold in order to reduce or even eliminate the risk of skin irritation or burns.

Prior art is known from US 9724533 B1 disclosing a TMS device for treatment of neurological disorders comprising at least one capacitor, a charging circuit and a magnet coil.

It is an objective of the present invention to provide a coil arrangement and a device with such a coil arrangement for TMS, which improve or even eliminate one or more of the aforementioned drawbacks. In particular, it is an objective of the present invention to provide a coil arrangement or coil design being manufacturable, providing low enough local power density in the wires, and concurrently a favorable focality and penetration-depth for TMS.

The objective is solved by a coil arrangement for transcranial magnetic stimulation, TMS according to a first aspect, comprising a first coil having a first plurality of windings, the first plurality of windings comprising at least an inner winding and an outer winding. The coil arrangement further comprises a second coil having a second plurality of windings, the second plurality of windings comprising at least an inner winding and an outer winding. The inner winding may be an innermost winding. The first and second coils are arranged adjacent to each other along a first predetermined surface, wherein the outer winding surrounds at least partially the inner winding of the respective first and second coils along the first predetermined surface. Moreover, adjacent sections of the outer windings of the first and second coils are at least partially arranged parallel to each other. The coil arrangement may be or comprise a coil design and/or a coil form.

Magnetic fields produced by the first and second coils when provided with a current appear as the sum of the individual coils' magnetic fields. By the provided specific design of the coil arrangement with the parallel sections, the magnetic fields can be leveraged to produce a specific spatial magnetic field distribution having a local power density, focality and precision similar or even better compared to one large coil. At the same time, such a design can be physically realized and manufactured. Furthermore, due to the coil arrangement comprising at least the first and second coils with the parallel sections, the heat generated by the coil arrangement is distributed by the first and second coils thereby reducing local heat peaks. The parallel sections optimally balance resistive losses in each coil with the electrical fields generated by the wire segments of said coils, thus producing the best possible electrical field at the desired location for the lowest possible power dissipation. The proposed design thus reduces power requirements, heating and optimizes the electrical field generated. In addition, the proposed design improves spatial resolution of transcranial magnetic stimulation and comfort while reducing skin burns with transcranial magnetic stimulation.

The coil arrangement, in particular the first and second coils of the coil arrangement may be made by winding a wire. Said wire may be a Litz wire. The wire, in particular the Litz wire may comprise a plurality of strands.

The first and second coils of the coil arrangement may be electrically conductive.

Numbers of the first and second pluralities of windings may be identical or different from each other.

The adjacent sections of the outer windings of the first and second coils are at least partially arranged parallel to each other. Since the first and second coils are arranged adjacent to each other, a section of the outer winding of the first coil is adjacent to a section of the outer winding of the second coil.

A section of the inner winding adjacent to the parallel section of the outer winding of the respective first and/or second coils may be at least partially arranged parallel to said parallel section of the outer winding. Due to the outer winding surrounding the inner winding, the parallel sections of the inner windings may be shorter than the parallel sections of the outer windings.

The first and/or second plurality of windings may comprise at least one intermediate winding arranged between the inner winding and the outer winding of the respective coil, wherein a section of the intermediate winding adjacent to the parallel section of the outer winding of the respective first and/or second coil is at least partially arranged parallel to said parallel section of the outer winding. As an option, the section of the inner winding adjacent to the parallel section of the intermediate winding of the respective first and/or second coils may be at least partially arranged parallel to said parallel section of the intermediate winding. Moreover, the parallel section of the intermediate winding may be shorter than the parallel section of the outer winding, but longer than the parallel section of the inner winding.

The parallel sections of the first coil may have a first predetermined spacing between each other along a spacing direction extending perpendicular to the parallel sections. In addition or as an alternative, the parallel sections of the second coil may have a second predetermined spacing between each other along the spacing direction. In addition or as an alternative, the parallel sections of the outer windings of the first and second coils may have a third predetermined spacing between each other along the spacing direction. The first, the second and/or the third predetermined spacings may be identical and/or different from each other. Thus, the first and second predetermined spacings may be identical, while the third predetermined spacing is different from the first and second predetermined spacings. In addition or as an alternative, adjacent parallel sections of the first coil and/or second coil may be in contact with each other or abut to each other. In addition or as an alternative, the parallel sections of the outer windings of the first and second coils may be in contact with each other or abut to each other. Accordingly, the parallel sections of the first coil and/or second may have no spacing between each other. At least said contacting and/or abutting parallel sections of the first and/or second coils may have an electrically insulating sleeve. The first, the second and/or the third predetermined spacings may be between 0 mm (in contact/abutting) to 10 mm. The first to third spacings may provide an optimal route of the windings of the wire forming the first and second coils. Furthermore, the first to third spacings prevent too sharp bends in the coil that would lead to manufacturing problems like broken wires.

Further sections of the windings of the first and/or second plurality of windings may be connected to the parallel sections and the further sections, in particular adjacent further sections of the respective coils may have different spacings between each other. As an option, the different spacings of the adjacent further sections may increase and/or decrease, in particular increase from the inner winding to the outer winding of the first and/or second coils. The different spacings may range from 0.2 mm to 2 mm, in particular 0.3 mm to 1.7 mm, advantageously from 0.4 mm to 1.4 mm. Thereby the coil design can be further optimized for manufacturing as well as usage thereof.

The further sections may have respective end sections connected to the parallel sections, wherein said end sections may have a predetermined curvature radius. The curvature radius of the windings of the first and/or second coils may at least partially be identical and/or different from each other. The curvature radius may range from 5 mm to 30 mm, in particular 8 mm to 25 mm, advantageously from 10 mm to 20 mm. The curved end sections may further reduce heat peaks. The curvature radius may be selected based on a material of the wire forming the first and second coils such that chances for broken wires and/or cable breaks are reduced or even eliminated.

The first and/or second coils may be at least partially arch-shaped, triangular-shaped, D-shaped, square-shaped, figure-of-eight-shaped if viewed together, helically-shaped and/or rectangular-shaped. The different shapes may be shaped along the first predetermined surface. Thus, the different shapes may be shapes as seen from a top view on the first predetermined surface. Depending on the first predetermined surface, one shape or a combination of the aforementioned shapes may be chosen to achieve an optimal arrangement on the first predetermined surface. Also, by choosing the shape, other components of a device comprising the coil arrangement may beneficially arranged in said device, e.g. next to the coil arrangement. Besides the parallel sections, the design of the coil arrangement can thus be chosen based on the arrangement and/or use thereof.

The first predetermined surface may be planar shaped, at least partially arch-shaped, ellipsoidal, cylindrical and/or arbitrarily curved. As an option, the first predetermined surface may form at least partially a head shape of a human being, in particular of a human being to be treated with transcranial magnetic stimulation. The first predetermined surface may advantageously be formed similar or identical to the head shape or a model thereof thereby reducing a spacing between the coil arrangement and the skin of the human. The coil arrangement may be formed such that the first and second plurality of windings, in particular all of the windings of the first and second plurality are arranged on the first predetermined surface.

The first and second coils may be formed by a single wire or two respective wires. The single wire may be wound to form the first and second coils. As an alternative, each of the first and second coils is produced by winding a respective wire. In other words, one wire is wound for both coils or two wires are wound for two coils. The two respective wires may each be wound to form the respective coil. The single wire may form single conductive line or circuit. The first and second coils may form two respective conductive lines or circuits. Accordingly, current provided to one of the first and second coils may also be provided to the other one of the first and second coils, since they are formed via the single conductive line. When the first and second coils are formed by the two wires, two separate circuits may be formed. The single wire may not be a single wire during production and end sections of the first and second coils may be connected, in particular via a bridging and/or connecting section as mentioned later, thereby forming a single wire or single conductive line or circuit.

The first and second coils may be formed by the single wire such that sections, in particular end sections of the inner windings of the first and second coils are connected by the bridging section of the single wire. As an option, the bridging section may be arranged opposite the first predetermined surface, in particular on a second predetermined surface. Thus, the coil arrangement is arranged at least partially between the first predetermined surface and the bridging section when viewed from a cross sectional view. The end sections may be sections of the first and second coils which may not represent a real physical end in case of the single wire, but merely define that it is an end part of the first or second coil. The bridging section may at least partially be formed straight and/or arch shaped, in particular viewed from the top view. A mirror axis extending parallel to the parallel sections may be arranged between the first and second coils, in particular between the outer windings of the first and second coils, when viewed from a top view on the first predetermined surface. A position of the end section of the first coil may be mirrored by the mirror axis and correspond to a position of the end section of the second coil. Accordingly, the bridging section may at least partially extend perpendicular to the parallel axis when connecting the end sections of the first and second coils. As an alternative, the positions of the end sections of the first and second coils may be different. Accordingly, when viewed from a top view, the bridging section may intersect the parallel axis at a predetermined angle, wherein the predetermined angle is different to 90° and/or excludes 90°. The predetermined angle may be determined depending on the arrangement of the end sections. Thus, the predetermined angle may be 20 to 40°, in particular 30°. The end sections of the inner windings of the first and second coils may be connected by the bridging section such that an angle between the end section of the respective coil and the bridging section is different from a perpendicular angle and/or may be 20 to 40°, in particular 30°. By choosing the predetermined angle different from 90°, kinking of the single wire may be avoided.

In addition or as an alternative, the first and second coils may be formed by winding the single wire, wherein the first and second plurality of windings each comprise an outermost winding, said two outermost windings being connected by the connecting section extending at least partially between and parallel to the adjacent sections of the outer windings. Accordingly, the connecting section may connect respective end sections of the outer windings of the first and second coils. The coil arrangement may have at least one of the bridging section and the connecting section.

The outermost winding may be the outer winding or may surround at least partially the outer winding of the respective coil.

The single wire may have at least partially a circular cross section. In addition or as an alternative, the single wire may have a square and/or rectangular cross section. A length along a length direction extending perpendicular to the spacing direction, a width along the spacing direction and a height extending perpendicular to the spacing and length direction of the respective cross section may respectively be selected. A length along the length direction and a height along the height direction of the square and/or rectangular cross section may have a ratio of 1:1 (square) to 1:3 (rectangular). Wires with a rectangular cross section may better fill the available volume used by the coil arrangement with the material of the wire, in particular copper, resulting in a more efficient use of space. Features related to the single wire may be implemented as features for a wire forming a coil of the coil arrangement. Thus, in case two wires are used for forming the first and second coils, the features relating to the single wire may be implemented as features of at least one of the two wires.

The singe wire may comprise a plurality of Litz strands, each strand, as an option, having a thickness between 0.01 to 0.3 mm, in particular between 0.07 mm to 0.15 mm, advantageously 0.10 mm.

An inner area formed by an inner circumference of the inner winding of the first and/or second coil may have a width along the spacing direction and/or a length along the length direction. In addition or as an alternative, the inner area may be at least partially arch-shaped, triangular-shaped, D-shaped, square-shaped, helically-shaped and/or rectangular-shaped. The inner area may be determined based on at least one of a Litz strand diameter, a TMS pulse length, an overall coil size of the coil arrangement, a Litz wire mechanical stiffness and/or pliability. The inner area may have an area of at least 2 to 3 cm x 2 to 3 cm along the spacing and length direction and/or an area of 400 to 900 mm², in particular 500 mm².

When viewed along the spacing direction from one inner area of one of the first and second coils to the other of the first and second coils, the parallel sections may be formed between said inner areas.

The coil arrangement may be potted in high-performance epoxy to reduce noise and increase stability and longevity of the coil arrangement by providing reduced mechanical stress and ensure stable curvature. In addition, the epoxy may act flame retardant and improve the thermal aspect and the electrical insulation of the coil arrangement. The coil arrangement may thus be potted using potting material with high thermal conductivity to improve dissipation of heat from the coils. The material may be 834HTC epoxy, in particular 834HTC-A epoxy.

In addition or as an alternative, the coil arrangement may further comprise at least one supporting section to support the coil arrangement, in particular one or more adjacent windings of the first and/or plurality of windings. The supporting section may comprise or be a plastic support sections. The at least one supporting section may be arranged between two adjacent windings of the first and/or second plurality of windings. The at least one supporting section may contact and/or abut at least partially against the two adjacent windings. The at least one supporting section may at least partially be formed based on the arrangement of the two adjacent windings. Between two adjacent windings of the first and/or second plurality of windings, one or more supporting sections may be provided. The plurality of supporting sections may at least partially contact and/or abut to one or more adjacent supporting sections. The at least one supporting section may be arranged between the adjacent windings of the first and/or second plurality of windings, excluding the parallel sections of the first and/or second plurality of windings. Thus, no supporting sections may be provided between the parallel sections. At least one outer supporting section may be provided for supporting the outer winding of the first and/or second plurality of windings. One outer supporting section may be provided which supports the outer windings of the first and second windings, which, as an option, may be integrally formed. Accordingly, an outer casing supporting the coil arrangement may be provided. The at least one supporting section may improve the longevity and/or thermal aspects of the coil arrangement.

The first and second coils may be defined as a first pair of coils. The coil arrangement may comprise or consist of the first pair of coils. As an alternative, the coil arrangement may comprise the first and at least a second pair of coils. The second pair of coils may comprise or consist of two coils. Said second pair of coils may be formed similar, in particular identical to the first pair of coils. The first and second pairs of coils may be stacked on each other. The first and second pairs of coils may be stacked such that the second pair of coils is arranged on the second predetermined surface opposite the first predetermined surface. The first and second predetermined surfaces may have a similar, in particular an identical shape. The first and second pairs of coils may be stacked such that the second pair of coils at least partially, in particular fully overlaps the first pair of coils. A number of pairs of coils may be chosen based on at least one of a TMS treatment and dimensions of a housing incorporating the coil arrangement. The number of pairs of coils may be 5 to 10, in particular 7. Features relating to the first predetermined surface may be implemented as features of the second predetermined surface.

Features of the first and second coils and thus the first pair of coils may also be implemented as features of the coils of the second pair of coils and any further pair of coils. The second pair of coils and any further pair of coils may each consist of two coils, similar to the first pair of coils consisting of the first and second coils. Accordingly, the coil arrangement may comprise one more layers, each layer consisting of one pair of coils.

The first pair of coils may form a first circuit and the second pair of coils may from a second circuit. The first and second circuits may not be connected with each other. Accordingly, each of the first and second circuits needs to be provided with a respective current in order to generate a respective magnetic field. As an alternative, the first and second pairs of coils may be connected to each other to form a common circuit. The first and second pairs of coils may be connected such that a third coil of the second pair of coils stacked on the first coil of the first pair of coils has a similar, in particular identical current flow direction. The same applies for a fourth coil of the second pair of coils and the second coil of the first pair of coils, which are stacked on each other. The first and second pairs of coils are connected such that the second and fourth coils have a similar, in particular identical current flow direction.

As an alternative, the first and second pairs of coils may be connected such that the first and third coils have respective different current flow directions and/or the second and fourth coils have respective different current flow directions. Based on the design and/or shape of the respective coils, a current flow direction may be clockwise or counter-clockwise.

If three or more pairs of coils are comprised by the coil arrangement, at least two of the first to third pairs of coils may be connected with each other to form a common circuit. In addition or as an alternative, least one of the first to third pairs of coils may form an individual circuit.

The at least second pair of coils may be formed by the same single wire as the first pair of coils. As an alternative, the two coils of the at least second pair of coils may be formed by a second single wire. Accordingly, at least two layers may be formed via the single wire and/or at least one layer may be formed by a respective wire.

The coil arrangement may be provided with a (electric) current at at least two sections of the coil arrangement such that the provided current can flow through the coil arrangement to generate a magnetic field, in particular for TMS. In particular, one of the two sections may be a section of the first coil and the other one of the two sections may be a section of the second coil. If two or more layers are connected with each other, said common circuit has at least said two sections for providing current. Said sections may be sections configured to be electrically coupled to a power source providing the current. Said sections may be end sections of the first and second coils or respective coils of the coil arrangement.

The objective is solved by a device for transcranial magnetic stimulation according to a second aspect, comprising a coil arrangement according to the second aspect.

The device may comprise a controller for controlling a current supplied to the coil arrangement and/or an energy source for providing the current. The device may comprise or be a casing accommodating the coil arrangement therein. The device may be a headset wearable by a user, in particular a patient.

Preferred embodiments are explained by way of example with reference to the enclosed drawings.
- Figs. 1 and 2: show a top view of a coil arrangement according to a first embodiment of the invention;
- Fig. 3: shows a top view of a coil arrangement according to a second embodiment of the invention;
- Figs. 4 and 5: show a perspective view of the coil arrangement according to the second embodiment of the invention;
- Fig. 6: shows a top view of a coil arrangement according to a third embodiment of the invention;
- Fig. 7: shows a top view of a coil arrangement according to a fourth embodiment of the invention; and
- Fig. 8: shows a device comprising a coil arrangement of any of the first to fourth embodiments.

In the drawings, identical or essentially functionally identical or similar elements are designated with the same reference signs.

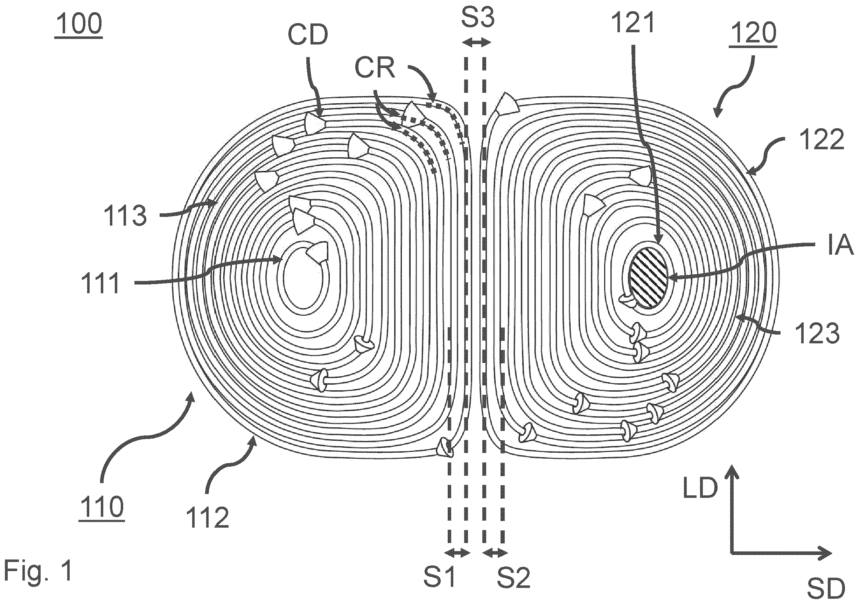

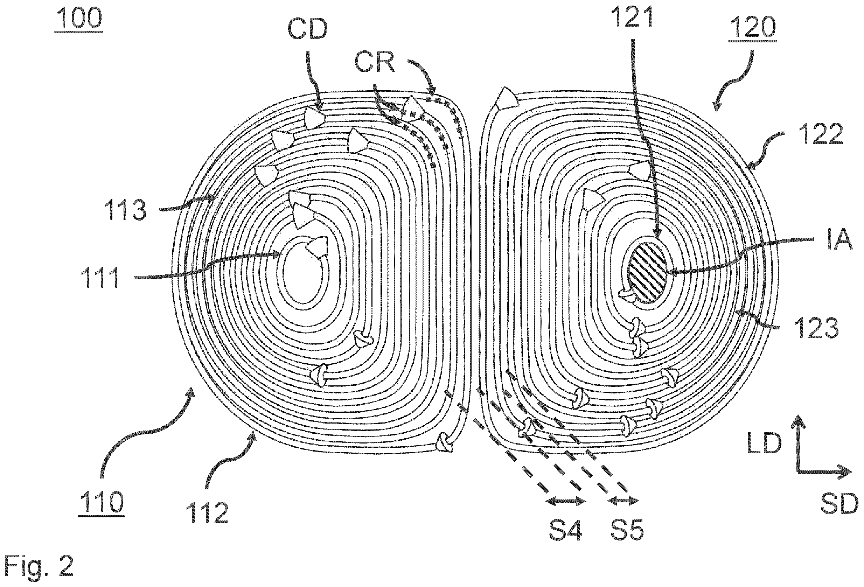

Figs. 1 and 2 show a first embodiment of a coil arrangement 100 transcranial magnetic stimulation, TMS. The arrangement 100 comprises a first coil 110 having a first plurality of windings, the first plurality of windings comprising at least an inner winding 111 and an outer winding 112. Furthermore, the arrangement 100 comprises a second coil 120 having a second plurality of windings, the second plurality of windings comprising at least an inner winding 121 and an outer winding 122. As illustrated in Figs. 1 and 2, each of the first and second plurality of windings of the first and second coils 110, 120 comprises a plurality of intermediate windings 113, 123 arranged between the outer and inner windings 111, 121, 112, 123.

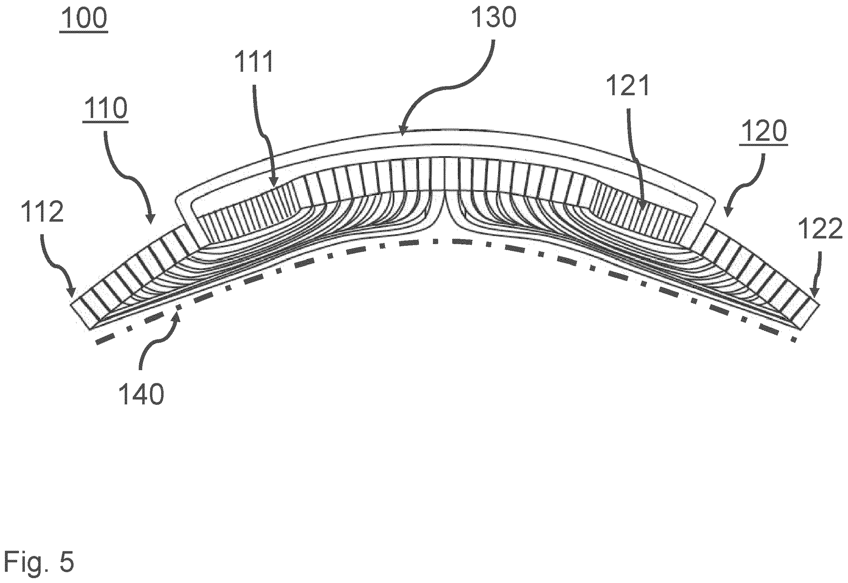

The first and second coils 110, 120 are arranged adjacent to each other along a first predetermined surface 140 (see e.g. Fig. 5), wherein the outer winding 112, 122 surrounds at least partially the inner winding 111, 121 of the respective first and second coils 110, 120 along the first predetermined surface. Also, adjacent sections of the outer windings 112, 122 of the first and second coils 110, 120 are at least partially arranged parallel to each other. As illustrated in Figs. 1 and 2, the intermediate windings 113, 123 adjacent to each other and the outer winding of the respective coil 110, 120 also comprise sections which are at least partially parallel to the outer winding 112, 122.

Figs. 1 and 2 further illustrate that the parallel sections of the first coil 110 have a first predetermined spacing S1 between each other and that the parallel sections of the second coil 120 have a second predetermined spacing S2 between each other. The first and second predetermined spacings S1 and S2 are shown as identical to each other. Moreover, a third predetermined spacing S3 is shown, which is a spacing between the two parallel sections of the outer windings 112, 122 of the first and second coils 110, 120. The third predetermined spacing S3 is also shown as identical to the first and second predetermined spacings S1, S2. However, the invention is not limited thereto, the first to third predetermined spacings S1-S3 may at least partially different from each other. The predetermined spacings S1-S3 are spacings along a spacing direction SD, which extends perpendicular to the parallel sections of the first and second coils 110, 120. Also shown is a length direction LD, which is parallel to the parallel sections of the first and second coils 110, 120.

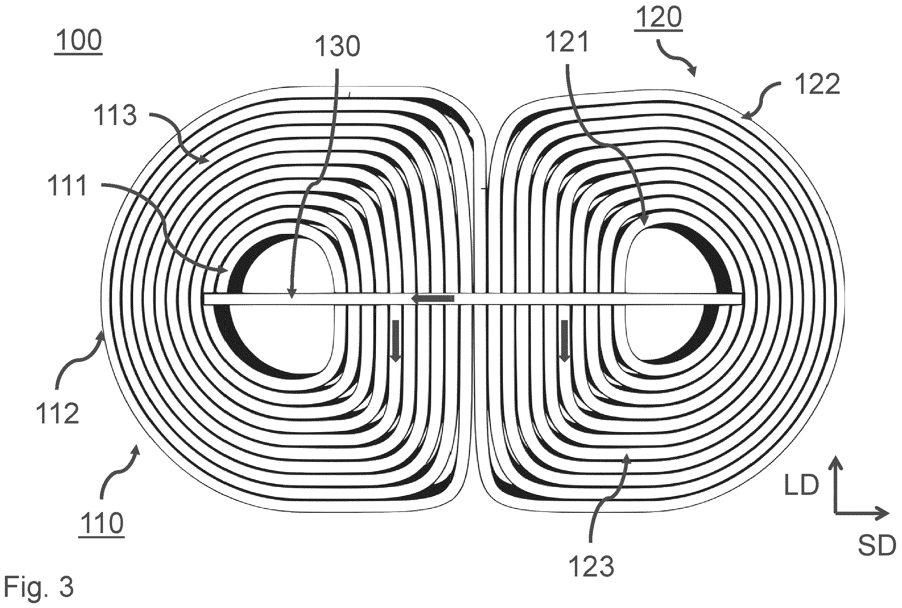

The first predetermined surface 140 may have a length along the length direction LD and a width along the spacing direction SD. However, the first predetermined surface 140 may alternatively be arch-shaped as shown in Figs. 3 to 5 or arbitrarily curved as required to bring the coil arrangement as close to a body, in particular a head of a patient as needed.

Figs. 1 and 2 further illustrate a current flow direction through the first and second coils 110, 120 and their plurality of windings indicated via arrows of the single windings. As shown in Figs. 1 and 2. The current flow direction of the first coil 110 is clockwise and the current flow direction of the second coil 120 is counter-clockwise. This could be inverted as well. By providing current through the coil arrangement 100, in particular the first and second coils 110, 120, respective magnetic fields are generated by the first and second coils 110, 120, which may partially overlap.

Furthermore, as shown in Figs. 1 and 2, an inner area IA defined by the inner winding is left free of wires and windings. By increasing the inner area IA, central heating effects during TMS can be significantly reduced. The inner area may be designed based on at least one of the following parameters: material of the wire forming the coil arrangement 100, the dimensions of the wire or wires, expected currents to be provided to the coils 110, 120 and frequency of the currents.

As found by the inventors, it is advantageous to wind the wire forming the coils 110, 120 such that end sections of the windings connected to the parallel sections have a predetermined curvature radius CR. Compared to the round curvature radius CR, formed edges with, e.g., an angle of 90° may form areas with heat peaks. Also, the coils 110, 120 with the curvature radius CR are more easily manufactured compared to the previously mentioned edged-shaped coils.

Fig. 2 further shows fourth and fifth predetermined spacings S4, S5. Further sections of the coils 110, 120 connected to the parallel sections may be wound such that the predetermined spacings between adjacent windings decreases from the outer winding 112, 122 to the inner winding 111, 121 of the respective coils 110, 120. Therefore, spacings of the further adjacent sections of the coils 110, 120 may be different from each other.

Figs. 1 and 2 do not show physical end sections of the coils 110, 120 and connections for providing current to them for illustrative purposes. As will be apparent also from the second embodiment of the present invention, the inner, intermediate and/or outer windings 111-113, 121-123 may be connected with each other and/or with another current providing energy source in order to provide current to the coil arrangement 100 for TMR.

Figs. 3 to 5 show perspective views of a coil arrangement 100 according to a second embodiment of the present invention with a first predetermined surface 140 being arch-shaped. Furthermore, a bridging section 130 connects sections, in particular end sections of the inner windings 111, 121. The bridging section 130 is arranged opposite the first predetermined surface and is arch-shaped and may be straight when viewed from the top view as illustrated in Fig. 3. However, the invention is not limited thereto. Since the two coils 110, 120 consist of winding a single wire, the bridging section 130 may be differently arranged. That is, when viewed in a two dimensional coordination system with the length and spacing direction, the position of the end section of the first coil 110 may be mirrored by an axis along the length direction and arranged between the first and second coils 110, 120. As shown in Fig. 3, the position of the end section of the first coil 110 is mirrored and matches with a position of the end section of the second coil 120. As an alternative, the positions of the end sections of the first and second coils 110, 120 may be different from each other. Furthermore, arrows indicate a flow direction of a current provided to the first and second coils 110, 120.

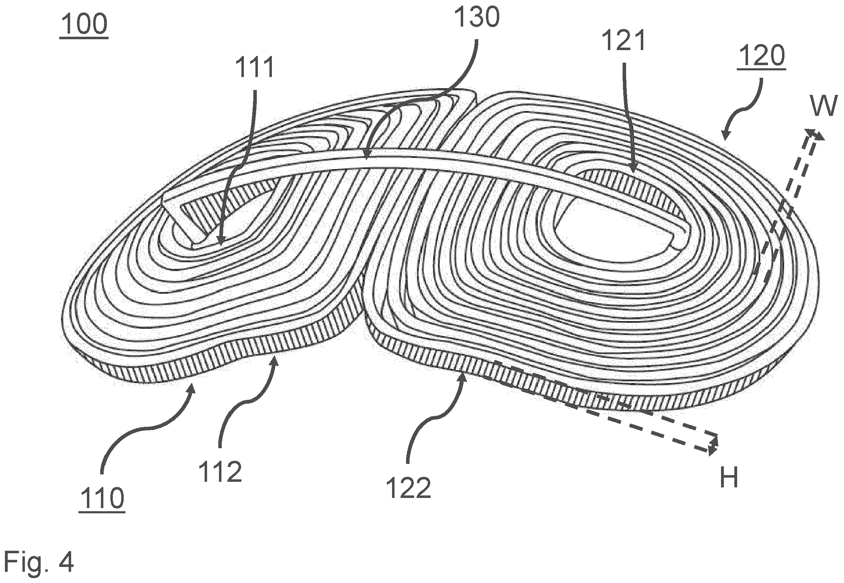

Furthermore, a single wire is wound such that the first and second coils 110, 120 and the bridging section 130 are formed. As an alternative, the first and second coils 110, 120 are formed by two individual wires. The single wire or the two wires may be a Litz wire or wires comprising a plurality of Litz strands. The single wire or the two wires may have a circular cross section. As shown in Fig. 4 and 5, the single wire has rectangular cross section. The single wire or the two wires may have a predetermined height H along a height direction and a predetermined width W along the spacing direction SD, wherein the height direction is perpendicular to the spacing and length directions SD, LD. In particular, the height of the single wire may be greater than the width thereof.

The coil arrangements 100 according to the first and second embodiments are D-shaped. That is, the first coil 110 is mirrored by a symmetry axis which extends parallel to the parallel sections of the first coil 110, when seen from a top view. When seen in the perspective view, the first coil 110 is mirrored by a symmetry plane extending along the symmetry axis and another axis perpendicular to the symmetry axis. Said another axis may be perpendicular to the length direction LD and the spacing direction SD.

In addition, the outer windings 112, 122 may be defined as outermost windings and/or the inner windings 111, 121 may be defined as innermost windings.

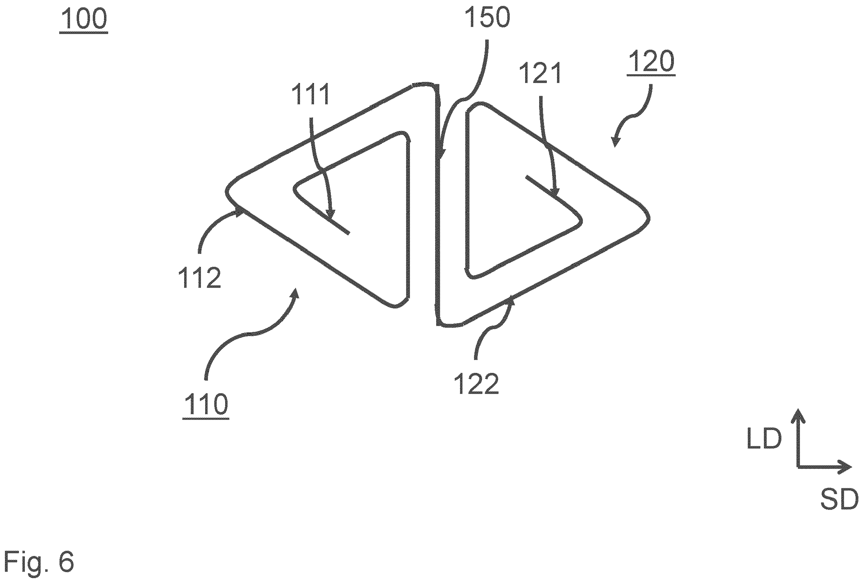

Fig. 6 shows a third embodiment of the coil arrangement 100 having a triangular shape. As illustrated in Fig. 6, the outermost windings of the first and second coils 110, 120 have or share a connecting section 150, wherein said connecting section 150 is arranged at least partially parallel to the parallel section of the first and second coils 110, 120. Accordingly, an electric connection between the first and second coils 110, 120 may be formed by the connecting section 150. Also, the triangular shaped arrangement 100 shows that additional adjacent sections of the first and second coils 110, 120 are at least partially parallel to each other.

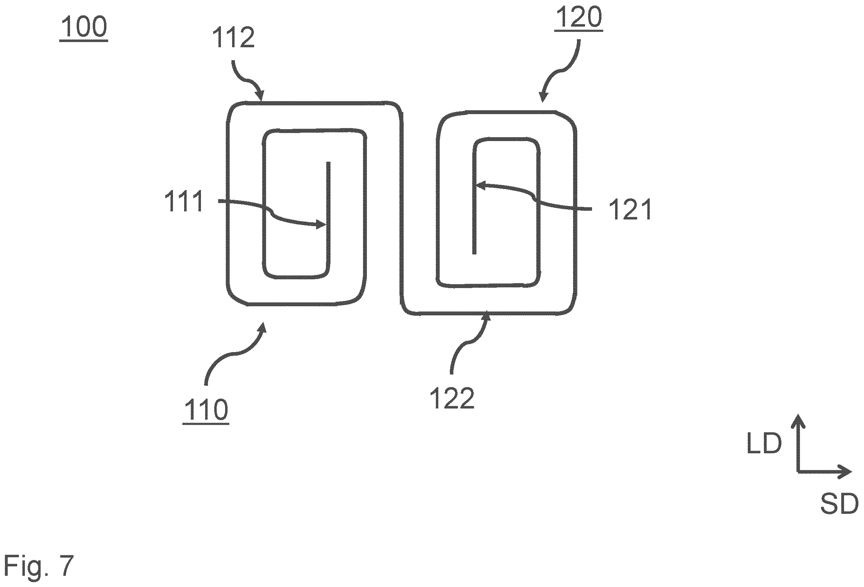

Fig. 7 shows a fourth embodiment of the coil arrangement 100 having a square- or rectangular-shape. Similar to the arrangement 100 according to the third embodiment, the arrangement 100 of the fourth embodiment has a connecting section connecting the first and second coils 110, 120 and also shows additional adjacent parallel sections.

The coil arrangements 100 according to the first to fourth embodiments have in common that the adjacent parallel sections have the first and second predetermined spacings S1, S2, while the spacings of the further sections of the windings of the first and second coils 110, 120 may be different or may even decrease from the outer winding 112, 122 to the inner winding 111, 121. As an alternative, adjacent parallel sections of at least one of the first and second coils may be in contact and/or abutting each other. In addition or as an alternative, the parallel sections of at least one of the first and second coils may be in contact and/or abutting the connecting section 150.

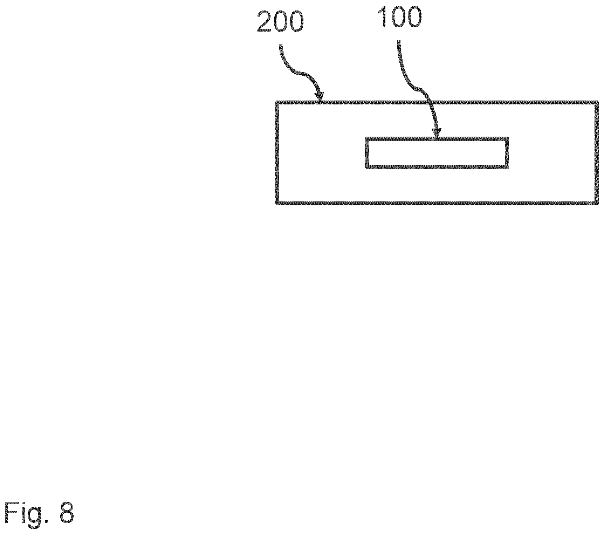

Fig. 8 shows a device 200 for transcranial magnetic stimulation comprising a coil arrangement 100 according to any of the first to fourth embodiments. The device 200 may be a headset wearable by a user, in particular a patient to be treated with TMS.

### REFERENCE SIGNS

- 100: coil arrangement
- 110: first coil
- 111: inner winding
- 112: outer winding
- 113: intermediate winding
- 120: second coil
- 121: inner winding
- 122: outer winding
- 123: intermediate winding
- CD: current flow direction
- SD: spacing direction
- LD: length direction
- CR: curvature radius
- IA: inner area
- S1: first predetermined spacing
- S2: second predetermined spacing
- S3: third predetermined spacing
- S4: spacing
- S5: spacing
- 130: bridging section
- 140: first predetermined surface
- 150: connecting section
- H: height of the single wire
- W: width of the single wire
- 200: device comprising a coil arrangement

## Claims

1. Coil arrangement (100) for transcranial magnetic stimulation, comprising:
a first coil (110) having a first plurality of windings, the first plurality of windings comprising at least an inner winding (111) and an outer winding (112);
a second coil (120) having a second plurality of windings, the second plurality of windings comprising at least an inner winding (121) and an outer winding (122),
wherein the first and second coils (110, 120) are arranged adjacent to each other along a first predetermined surface (140),
wherein the outer winding (112, 122) surrounds at least partially the inner winding (111, 121) of the respective first and second coils (110, 120) along the first predetermined surface (140),
wherein adjacent sections of the outer windings (112, 122) of the first and second coils (110, 120) are at least partially arranged parallel to each other.

2. Coil arrangement (100) according to claim 1,
wherein a section of the inner winding (111, 121) adjacent to the parallel section of the outer winding (112, 122) of the respective first and/or second coils (110, 120) is at least partially arranged parallel to said parallel section of the outer winding (112, 122).

3. Coil arrangement (100) according to claim 1 or 2,
wherein the first and/or second plurality of windings comprise at least one intermediate winding (113, 123) arranged between the inner winding (111, 121) and the outer winding (112, 122) of the respective coil (110, 120),
wherein a section of the intermediate winding (113, 123) adjacent to the parallel section of the outer winding (112, 122) of the respective first and/or second coil (110, 120) is at least partially arranged parallel to said parallel section of the outer winding (112, 122),
wherein, as an option, the section of the inner winding (111, 121) adjacent to the parallel section of the intermediate winding (113, 123) of the respective first and/or second coils (110, 120) is at least partially arranged parallel to said parallel section of the intermediate winding (113, 123).

4. Coil arrangement (110) according to any of the previous claims,
wherein the parallel sections of the first coil (110) have a first predetermined spacing (S1) between each other along a spacing direction (SD) extending perpendicular to the parallel sections, and/or
wherein the parallel sections of the second coil (120) have a second predetermined spacing (S2) between each other along the spacing direction (SD), and/or
wherein the parallel sections of the outer windings (112, 123) of the first and second coils (110, 120) have a third predetermined spacing (S3) between each other along the spacing direction (SD).

5. Coil arrangement (100) according to any of the previous claims,
wherein further sections of the windings of the first and/or second plurality of windings connected to the parallel sections have different spacings (S4, S5) between each other,
wherein, as an option, the different spacings (S4, S5) of the adjacent further sections increase and/or decrease from the inner winding (111, 1221) to the outer winding (121, 122) of the first and/or second coils (110, 120).

6. Coil arrangement (100) according to any of the previous claims,
wherein the first and/or second coils (110, 120) are at least partially arch-shaped, triangular-shaped, D-shaped, square-shaped, figure-of-eight-shaped, helically-shaped and/or rectangular-shaped.

7. Coil arrangement (100) according to any of the previous claims,
wherein the first (140) predetermined surface is planar shaped or at least partially arch-shaped, ellipsoidal-shaped and/or cylindrically shaped,
wherein, as an option, the first (140) predetermined surface forms at least partially a head shape of a human being, in particular of a human being to be treated with transcranial magnetic stimulation.

8. Coil arrangement (100) according to any of the previous claims,
wherein the first and second coils (110, 120) are formed by a single wire or two respective wires.

9. Coil arrangement (100) according to claim 8,
wherein the first and second coils (110, 120) are formed by the single wire,
wherein sections, in particular end sections of the inner windings (111, 121) of the first and second coils (110, 120) are connected by a bridging section (130) of the single wire,
wherein, as an option, the bridging section (130) is arranged opposite the first predetermined surface.

10. Coil arrangement (100) according to claim 8 or 9,
wherein the first and second coils (110, 120) are formed by the single wire,
wherein the first and second plurality of windings each comprise an outermost winding,
said two outermost windings being connected by a connecting section (150) extending at least partially between and parallel to the adjacent sections of the outer windings.

11. Coil arrangement (100) according to any of the previous claims,
wherein an inner area (IA) formed by an inner circumference of the inner winding (111, 121) of the first and/or second coil (110, 120) is at least partially arch-shaped, triangular-shaped, D-shaped, square-shaped, helically-shaped and/or rectangular-shaped.

12. Device (200) for transcranial magnetic stimulation, comprising a coil arrangement (100) according to any of the previous claims.
